# EUROPEAN PATENT APPLICATION

(11) **EP 4 775 132 A1**
(43) Date of publication of application: **15.07.2026**
(21) Application number: 24882820.4
(22) Date of filing: 23.10.2024
(51) Int. Cl.: A61B 5/00, A61B 5/024, A61B 5/1455

(54) **WEARABLE ELECTRONIC DEVICE**

(30) Priority: 24.10.2023 KR 20230142564; 28.12.2023 KR 20230195665
(71) Applicant: Samsung Electronics Co., Ltd., Suwon-si, Gyeonggi-do 16677 (KR)
(72) Inventor: KIM, Hongki, Suwon-si Gyeonggi-do 16677 (KR); KANG, Daegyu, Suwon-si Gyeonggi-do 16677 (KR); MIN, Eungi, Suwon-si Gyeonggi-do 16677 (KR); LEE, Jeehoon, Suwon-si Gyeonggi-do 16677 (KR)
(74) Representative: Appleyard Lees IP LLP
(86) International application number: PCT/KR2024/016198
(87) International publication number: WO 2025/089793

(57) **Abstract**

An electronic device according to an embodiment of the present disclosure may comprise: a housing including an outer housing portion and an inner housing portion which is disposed inside the outer housing portion and includes at least one protrusion protruding inward; a circuit board positioned in the housing; an optical element which is positioned in the housing at a position corresponding to the at least one protrusion and which is mounted on the circuit board; and a conductor electrically connecting the circuit board to the optical element. The inner housing portion may include a first coating layer disposed to surround the optical element and having a first light transmittance.

## Description

### [Technical Field]

An embodiment disclosed in this document relates to a wearable electronic device.

### [Background Art]

Recent electronic devices come in various form factors for user convenience purposes and in reduced size for easy carrying. For example, the electronic device may be provided in the form of a ring that may be worn on the user's finger. More interest is being paid to health, and so is technology capable of checking up the health condition.

Accordingly, electronic devices may include a sensor for measuring the user's biometric data and have been developed to measure and utilize various biometric signals using the sensor and provide various services for the user's health care or check on the user's health condition through measurement of various biometric signals.

The information may be provided as a related art for the purpose of helping understanding of the disclosure. No claim or determination is made as to whether any of the foregoing is applicable as background art in relation to the disclosure.

### [Detailed Description of the Invention]

### [Technical Solution]

An electronic device according to an embodiment of the disclosure may include a housing including an outer housing portion, and an inner housing portion disposed inside the outer housing portion and including at least one protrusion protruding in an inward direction, a circuit board positioned within the housing, an optical element positioned within the housing at a position corresponding to the at least one protrusion and mounted on the circuit board, and a conductor electrically connecting the circuit board and the optical element. The inner housing portion may include a first coating layer disposed to surround the optical element and having a first light transmittance.

An electronic device according to an embodiment of the disclosure may include a housing including an outer housing portion, and an inner housing portion disposed inside the outer housing portion, a circuit board positioned within the housing, an optical element positioned within the housing and mounted on the circuit board, and a conductor electrically connecting the circuit board and the optical element. The inner housing portion may include a first coating layer disposed to surround the optical element and having a first light transmittance, and a second coating layer disposed to surround the first coating layer and having a second light transmittance lower than the first light transmittance.

An electronic device according to an embodiment of the disclosure may include a housing including an outer housing portion, and an inner housing portion disposed inside the outer housing portion and including at least one protrusion protruding in an inward direction, a circuit board positioned within the housing, an optical element positioned within the housing at a position corresponding to the at least one protrusion and mounted on the circuit board, and a conductor electrically connecting the circuit board and the optical element. The inner housing portion may include a first coating layer disposed to surround the optical element and having a first light transmittance, and a second coating layer disposed to surround the first coating layer and having a second light transmittance lower than the first light transmittance.

### [Brief Description of Drawings]

The above-described aspects or other aspects, configurations and/or advantages of an embodiment of the disclosure may become clearer through the following detailed description with reference to the accompanying drawings.
FIG. 1 is a block diagram illustrating an electronic device in a network environment according to an embodiment of the disclosure.
FIG. 2 is a view illustrating usage examples of a wearable electronic device according to an embodiment of the disclosure.
FIG. 3 is a perspective view illustrating a wearable electronic device according to an embodiment of the disclosure.
FIG. 4 is a cross-sectional view illustrating a wearable electronic device according to an embodiment of the disclosure;
FIG. 5 is an exploded perspective view illustrating a wearable electronic device according to an embodiment of the disclosure.
FIG. 6A is a side cross-sectional view illustrating a wearable electronic device 200 according to embodiments of the disclosure.
FIG. 6B is an enlarged view illustrating at least one protrusion of the wearable electronic device 200 according to embodiments of the disclosure.
FIG. 7 is a side cross-sectional view illustrating a portion of a wearable electronic device according to embodiments of the disclosure.
FIG. 8 is a side cross-sectional view illustrating an inner housing portion of a wearable electronic device according to embodiments of the disclosure.
FIG. 9 is a view illustrating a circuit board according to embodiments of the disclosure.
FIGS. 10A and 10B are diagrams describing transmittance of a first coating layer and a second coating layer of an inner housing portion according to embodiments of the disclosure.
FIG. 11 is a side cross-sectional view illustrating an inner housing portion of a wearable electronic device in which both a light emitting element and a light receiving element are disposed according to embodiments of the disclosure.
FIG. 12 is a diagram describing transmittance of a first coating layer and a second coating layer of an inner housing portion according to embodiments of the disclosure.
FIG. 13 is a side cross-sectional view illustrating a portion of a wearable electronic device including an inner housing portion having substantially the same transmittance according to embodiments of the disclosure.
FIGS. 14A and 14B are diagrams describing transmittance of an inner housing portion according to embodiments of the disclosure.
FIG. 15 is a view illustrating a change in light quantity at a blood vessel portion according to a curvature of at least one protrusion and a separation distance between the at least one protrusion and user skin according to embodiments of the disclosure.
FIG. 16 is a side cross-sectional view illustrating distribution of light quantity entering an optical element according to embodiments of the disclosure.

Throughout the accompanying drawings, similar reference numbers may be allocated to similar portions, configurations, and/or structures.

### [Mode for Carrying out the Invention]

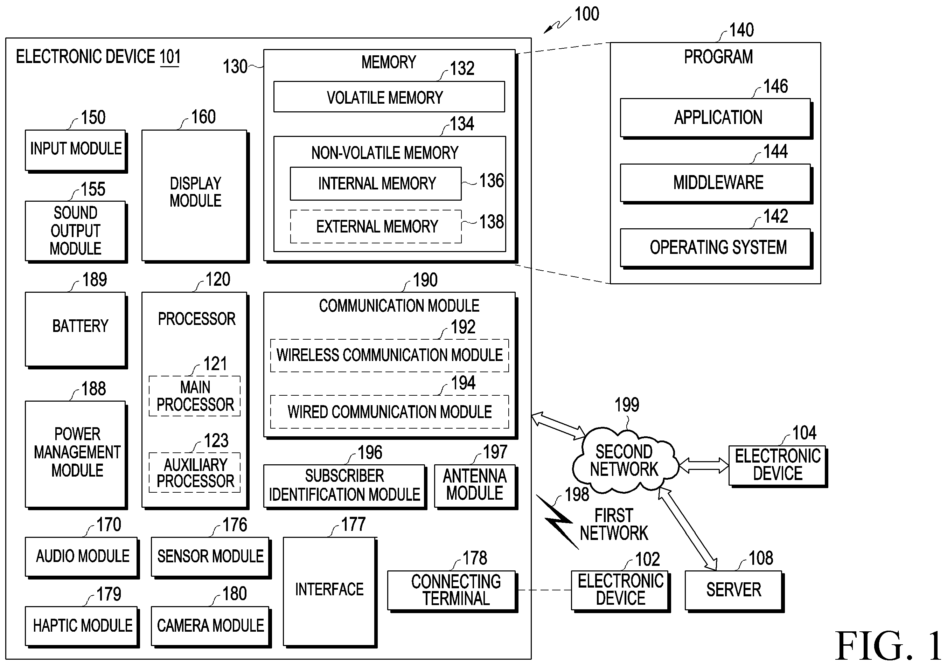

FIG. 1 is a block diagram illustrating an electronic device 101 in a network environment 100 according to various embodiments.

Referring to FIG. 1, the electronic device 101 in the network environment 100 may communicate with at least one of an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In an embodiment, at least one (e.g., the connecting terminal 178) of the components may be omitted from the electronic device 101, or one or more other components may be added in the electronic device 101. According to an embodiment, some (e.g., the sensor module 176, the camera module 180, or the antenna module 197) of the components may be integrated into a single component (e.g., the display module 160).

The processor 120 may execute, for example, software (e.g., the program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be configured to use lower power than the main processor 121 or to be specified for a designated function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. The artificial intelligence model may be generated via machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by other component (e.g., the processor 120) of the electronic device 101, from the outside (e.g., a user) of the electronic device 101. The input module 150 may include, for example, a microphone, a mouse, a keyboard, keys (e.g., buttons), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor configured to detect a touch, or a pressure sensor configured to measure the intensity of a force generated by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operation state (e.g., power or temperature) of the electronic device 101 or an external environmental state (e.g., the user's state), and then generate an electrical signal or data value corresponding to the detected state. According to an embodiment, the sensor module 176 may include, for example, a gesture sensor, a gyro sensor, an atmospheric pressure sensor, a magnetic sensor, an accelerometer, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or motion) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wiredly) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wiredly) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via a first network 198 (e.g., a short-range communication network, such as Bluetooth^{™}, wireless-fidelity (Wi-Fi) direct, or infrared data association (IrDA)) or a second network 199 (e.g., a long-range communication network, such as a legacy cellular network, a 5G network, a next-generation communication network, the Internet, or a computer network (e.g., local area network (LAN) or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device). According to an embodiment, the antenna module 197 may include one antenna including a radiator formed of a conductor or conductive pattern formed on a substrate (e.g., a printed circuit board (PCB)). According to an embodiment, the antenna module 197 may include a plurality of antennas (e.g., an antenna array). In this case, at least one antenna appropriate for a communication scheme used in a communication network, such as the first network 198 or the second network 199, may be selected from the plurality of antennas by, e.g., the communication module 190. The signal or the power may then be transmitted or received between the communication module 190 and the external electronic device via the selected at least one antenna. According to an embodiment, other parts (e.g., radio frequency integrated circuit (RFIC)) than the radiator may be further formed as part of the antenna module 197.

According to an embodiment, the antenna module 197 may form a mmWave antenna module. According to an embodiment, the mmWave antenna module may include a circuit board, a RFIC disposed on a first surface (e.g., the bottom surface) of the circuit board, or adjacent to the first surface and capable of supporting a designated high-frequency band (e.g., the mmWave band), and a plurality of antennas (e.g., array antennas) disposed on a second surface (e.g., the top or a side surface) of the circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. The external electronic devices 102 or 104 each may be a device of the same or a different type from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an Internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

The electronic device according to an embodiment of the disclosure may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. In connection to the description of the drawings, similar reference numerals may be used for similar or related components. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

An embodiment of the disclosure may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a compiler or a code executable by an interpreter. The storage medium readable by the machine may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program products may be traded as commodities between sellers and buyers. The computer program product may be distributed in the form of a machine-readable storage medium (e.g., compact disc read only memory (CD-ROM)), or be distributed (e.g., downloaded or uploaded) online via an application store (e.g., Play Store^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to an embodiment, each component (e.g., a module or a program) of the above-described components may include a single entity or multiple entities. Some of the plurality of entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

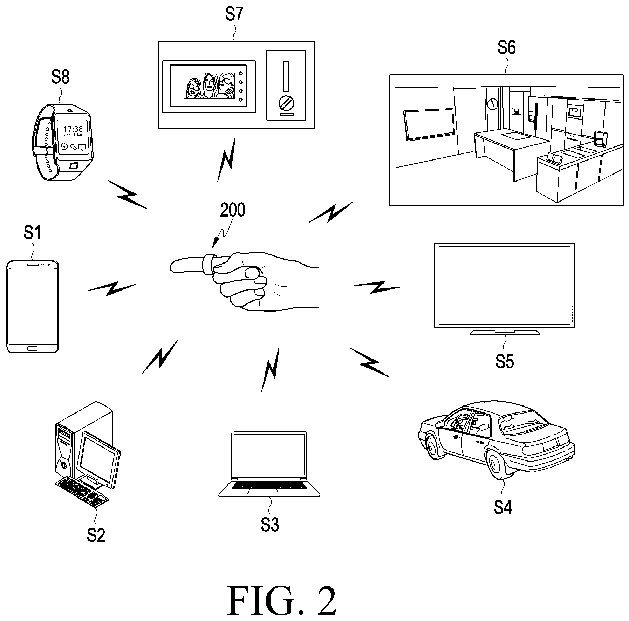

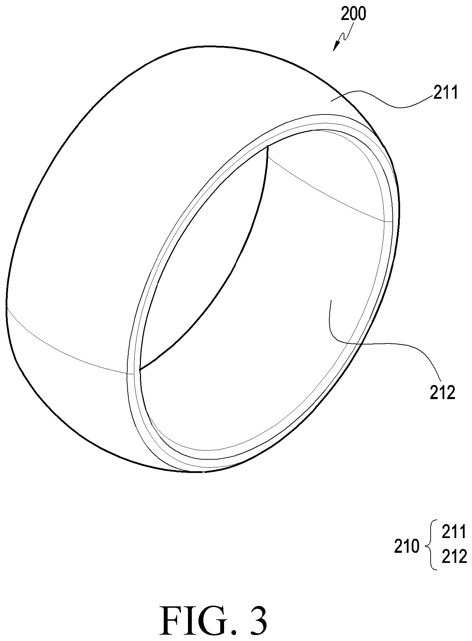

FIG. 2 is a view illustrating usage examples of a wearable electronic device according to an embodiment of the disclosure. The embodiment of FIG. 2 may be combined with the embodiment of FIG. 1 or the embodiments of FIGS. 3 to 17.

Referring to FIG. 2, a wearable electronic device 200 (e.g., the electronic device 101 of FIG. 1) may be configured to be wearable on the user's body. For example, the wearable electronic device 200 may be implemented as a wearable electronic device wearable on the user's finger. For example, the wearable electronic device 200 may be provided in the form of a ring that may be worn on the user's finger. The wearable electronic device 200 may be defined and/or referred to as a smart ring.

According to an embodiment, the wearable electronic device 200 may perform wireless communication with another electronic device (e.g., the electronic device 102 or 104 of FIG. 1) through a wireless communication network (e.g., the first network 198 or the second network 199 of FIG. 1). For example, the wearable electronic device 200 may perform wireless communication with another electronic device such as a smart phone S1, desktop/laptop computers S2 and S3, a car S4, a smart TV S5, indoor smart home devices S6, a tablet PC S7, or a smart watch S8. Wireless communication between the wearable electronic device 200 and another electronic device may be implemented as wireless communication via a short-range communication network (e.g., the first network 198 of FIG. 1) or a long-range communication network (e.g., the second network 199 of FIG. 1). For example, if a Bluetooth communication link is established between the wearable electronic device 200 and the electronic device to which the user wants to access, a message may be transferred between the two electronic devices, and the wearable electronic device 200 worn by the user may generate a command corresponding to each specific motion/gesture of the user's finger and transfer the command to the other electronic device. Motion sensors (e.g., the sensor module 176 of FIG. 1) such as an accelerometer, a gyroscope, or an electronic compass may be disposed in the wearable electronic device 200 to detect a user's finger movement/gesture or the like. When a message is received from another electronic device to the wearable electronic device 200, the electronic device 200 may notify the user of message reception using sound, vibration, a display screen, or lighting (e.g., a light emitting diode or a xenon lamp). To this end, the wearable electronic device 200 may include a sound module (e.g., the sound output module 155 of FIG. 1, or the audio module 170), a haptic module (e.g., the haptic module 179 of FIG. 1), or a display module (e.g., the display module 160 of FIG. 1). According to an embodiment, at least one of a sound module, a haptic module, or a display module may be omitted from the electronic device, or one or more other components may be output. Further, the wearable electronic device 200 may obtain biometric data (e.g., oxygen saturation) of a user and provide the biometric data to another electronic device.

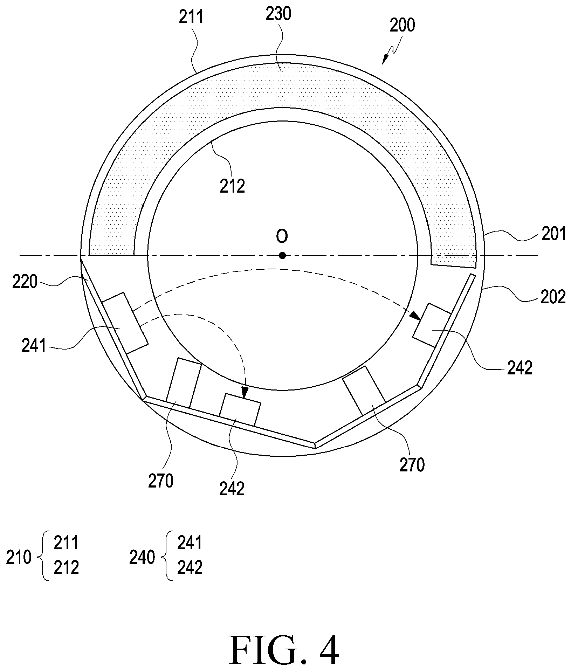

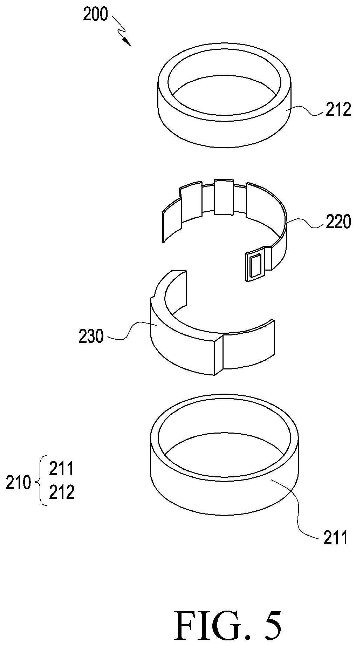

FIG. 3 is a perspective view illustrating a wearable electronic device according to an embodiment of the disclosure. FIG. 4 is a cross-sectional view illustrating a wearable electronic device according to an embodiment of the disclosure; FIG. 5 is an exploded perspective view illustrating a wearable electronic device according to an embodiment of the disclosure.

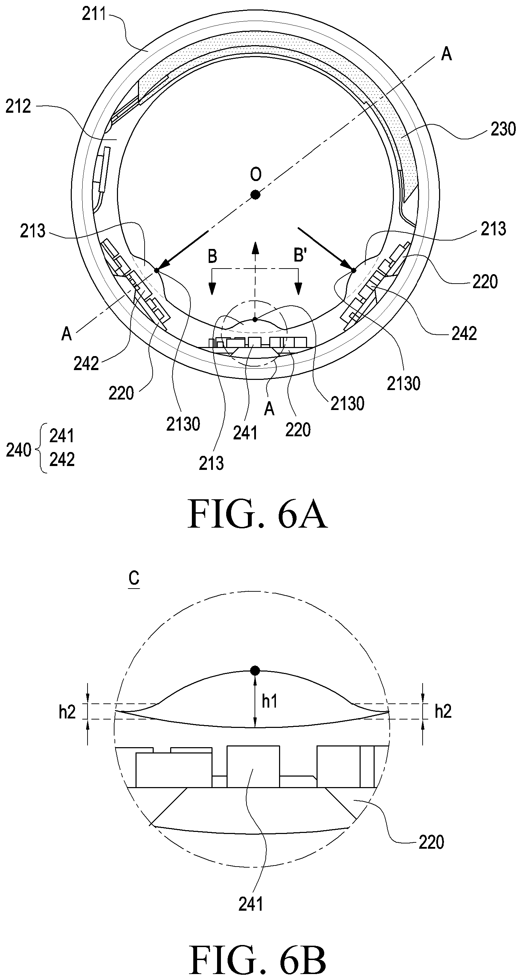

The embodiments of FIGS. 3 and 5 may be combined with the embodiments of FIGS. 1 to 2 or the embodiments of FIGS. 6 to 17. The configuration of the wearable electronic device 200 of FIGS. 3 to 5 may be identical in whole or part to the configuration of the electronic device 101 of FIG. 1 or the configuration of the wearable electronic device 200 of FIG. 2.

Referring to FIGS. 3 to 5, the wearable electronic device 200 may include a housing 210. The housing 210 may form the overall appearance of the wearable electronic device 200.

According to an embodiment, the housing 210 may have a ring shape (e.g., a finger ring shape). For example, the housing 210 may include an opening configured to receive a user's finger. For example, the housing 210 may include an opening configured to receive a user's wrist. For example, the opening may be defined as a hole implemented by the housing 210. However, the wearable electronic device 200 is not limited to the embodiment of the ring shape, and may be designed in various shapes (e.g., a box shape) that may be worn and/or fixed on a user's body (e.g., a finger, a wrist).

According to an embodiment, the housing 210 may include an outer housing portion 211 or an inner housing portion 212. The inner housing portion 212 may be coupled to the outer housing portion 211. For example, the inner housing portion 212 may be coupled to the outer housing portion 211 from inside the outer housing portion 211. According to an embodiment, the outer housing portion 211 and the inner housing portion 212 may be manufactured separately and assembled, or may be integrally formed. In an embodiment, the outer housing portion may be manufactured from a metal material and the inner housing portion may be formed by applying a liquid material to the inside of the outer housing portion and curing the liquid material. In an embodiment, a portion of the inner housing portion may be implemented by combining a portion manufactured from a metal material or synthetic resin with a portion formed by applying/curing a liquid material.

According to an embodiment, the outer housing portion 211 may include a material that may withstand external impact and/or scratches and implement design features. For example, the outer housing portion 211 may include at least one of titanium, stainless steel, or ceramic. The outer housing portion 211 may be colored or coated to implement a design.

According to an embodiment, the inner housing portion 212 may be a portion that touches the user's finger when the user wears the wearable electronic device 200. The inner housing portion 212 may be formed of a material such as a molding material, transparent plastic, or glass for sensing. For example, the inner housing portion 212 may be configured to be at least partially transparent. For example, the inner housing portion 212 may include a material through which light for measuring biometric data may be transmitted. At least a portion of the inner housing portion 212 may be manufactured from substantially the same or similar material as the outer housing portion 211. Further, at least a portion of the inner housing portion 212 may include a metal material for biometric data measurement.

According to an embodiment, the outer housing portion 211 and the inner housing portion 212 may be coupled to provide an inner space of the housing 210. Here, "the inner space of the housing 210" may be understood as a space separate from the opening for receiving a portion of the user's body. For example, various electrical/electronic components of the wearable electronic device 200 may be disposed and/or mounted in the inner space of the housing 210. For example, the housing 210 may receive various electrical/electronic components.

According to an embodiment, the wearable electronic device 200 may include a circuit board 220, a battery 230, and at least one optical module 240 disposed within the housing 210.

According to an embodiment, the circuit board 220 may be disposed in the inner space of the housing 210. The circuit board 220 may include at least one of a printed circuit board (PCB), a flexible printed circuit board (FPCB), or a rigid-flexible PCB (RF-PCB).

According to an embodiment, various electrical/electronic components may be disposed and/or mounted on the circuit board 220. For example, a processor (e.g., the processor 120 of FIG. 1), memory (e.g., the memory 130 of FIG. 1), a communication module (e.g., the communication module 190 of FIG. 1), or a sensor module (e.g., the sensor module 197 of FIG. 1, or the at least one optical module 240 of FIG. 4) may be mounted on the circuit board 220.

According to an embodiment, the circuit board 220 may include a plurality of circuit boards. For example, the plurality of circuit boards may be disposed according to the shape of the inner space of the housing 210 and may be electrically connected to each other. The circuit board 220 may include a flexible printed circuit board (FPCB). For example, the flexible printed circuit board may be at least partially bent according to the shape of the inner space of the housing 210. For example, the circuit board 220 may include a rigid circuit board and a flexible circuit board alternately disposed with respect to a circumferential direction (or, a peripheral direction) of the housing 210.

According to an embodiment, the battery 230 may be a device for supplying power to a component of the wearable electronic device 200 and may include a non-rechargeable primary cell, or a rechargeable secondary cell, or a fuel cell. The battery 230 may be integrally disposed inside the wearable electronic device 200 or may be detachably disposed from the wearable electronic device 200. According to an embodiment, the battery 230 may be formed as a single integrated battery or may include a plurality of separable batteries. The battery 230 may include a battery pack that is bent according to the shape of the inner space of the housing 210. The battery 230 may include a plurality of non-bendable battery packs of the housing 210. The battery 230 may include a battery pack that is bent and a plurality of non-bendable battery packs.

According to an embodiment, the circuit board 220 may be disposed in a second region 202 of the housing 210 (e.g., a lower region of the housing in the state illustrated in FIG. 4). According to an embodiment, the battery 230 may be disposed in a first region 201 (e.g., an upper region of the housing in the state illustrated in FIG. 4) rather than the second region 202 of the housing 210. For example, the housing 210 may be divided into a first region 201 above a reference line and a second region 202 below the reference line based on a virtual reference line passing through a center O, the battery 230 may be disposed in the first region 201, and the circuit board 220 may be disposed in the second region 202. The first region 201 and the second region 202 may have substantially the same size and shape. The first region and the second region may be substantially symmetrical with respect to the center O of the housing 210. However, the embodiment(s) of the disclosure is not limited thereto, and a region in which the circuit board 220 is disposed and a region in which the battery 230 is disposed may be defined differently from what is illustrated in the drawings according to the size or number of the battery(s) and circuit board(s).

According to an embodiment, the wearable electronic device 200 may include a power management module (e.g., the power management module 188 of FIG. 1) disposed on the circuit board 220.

According to an embodiment, the optical module 240 of the wearable electronic device 200 may be disposed within the housing 210 and electrically connected to the circuit board 220. According to an embodiment, the optical module 240 of the wearable electronic device 200 may include at least one light emitting module 241 and at least one light receiving module 242. According to an embodiment, the optical module 240 of the wearable electronic device 200 may be used as a sensor for obtaining (or measuring) at least one type of biometric data. For example, the at least one type of biometric data may include at least one of oxygen saturation information of a user or heart rate information of the user. For example, the sensor may include a photo plethysmography (PPG) sensor for oxygen saturation measurement or heart rate measurement.

According to an embodiment, the PPG sensor may include a light source (e.g., the at least one light emitting module 241) configured to emit light of two wavelength bands (e.g., a RED wavelength band or an infrared wavelength band). The PPG sensor may include a light receiving portion (e.g., the at least one light receiving module 242) configured to sense at least a portion of light reflected from or transmitted through a user's body portion (e.g., a finger, skin of the finger, or a blood vessel of the finger).

According to an embodiment, the at least one light emitting module 241 may emit light of substantially the same or different wavelengths to radiate light to a user's body portion (e.g., a finger, skin and/or blood vessel of the finger) for oxygen saturation measurement of the user. For example, the at least one light emitting module 241 may emit light of various bands, and may include at least one of a light emitting diode (LED), a laser diode, or a vertical cavity surface emitting laser (VCSEL). The at least one light emitting module 241 may be disposed and/or mounted on the circuit board 220. The at least one light emitting module 241 may be configured to sequentially (or repeatedly) emit light of different wavelength bands by dividing time.

According to an embodiment, the at least one light receiving module 242 may accumulate photocharge corresponding to an amount of light incident by being reflected from and/or transmitted through a user's body portion, and convert a bio-signal in the form of analog current according to the accumulated photocharge into a digital signal. For example, the light (or optical signal) obtained (or sensed) through the at least one light receiving module 242 may be converted through an analog to digital converter (ADC) and stored in memory or a sensor buffer. The at least one light receiving module 242 may include at least one of a photodiode (PD), a photo transistor, a charge-coupled device (CCD), or a complementary metal oxide semiconductor (CMOS). The at least one light receiving module 242 is not limited thereto and may include various elements capable of converting an incident optical signal into an electrical signal.

According to an embodiment, the at least one light receiving module 242 may be configured to receive at least a portion of light transmitted through a user's body portion or light reflected by at least a portion of the user's body portion. The at least one light receiving module 242 may receive light transmitted through a user's body portion, convert the transmitted light into an electrical signal, and transmit the electrical signal to a processor (e.g., the processor 120 of FIG. 1). According to an embodiment, the light receiving module 242 may be configured to receive light reflected by at least a portion of a user's body portion. The light receiving module 242 may receive light reflected from a user's body portion, convert the reflected light into an electrical signal, and transmit the electrical signal to a processor (e.g., the processor 120 of FIG. 1).

According to an embodiment, light emitted from the at least one light emitting module 241 may reach the at least one light receiving module 242 through an optical path. For example, the optical path may be a path transmitted through a user's body portion (e.g., a finger, skin of the finger, or a blood vessel of the finger), or may be a path reflected from the user's body portion. For example, at least a portion of the light emitted from a light emitting module may reach a light receiving module after being transmitted through a user body or reflected by the user body. The light receiving module may receive light transmitted through a user body or reflected by the user body and convert the light into an electrical signal, and the converted electrical signal may be used as data for determining user biometric data (e.g., oxygen saturation information or heart rate information of the user).

According to an embodiment, the wearable electronic device 200 may include at least one blocking member 270. The at least one blocking member 270 may include a material that absorbs or blocks at least a portion of light emitted from the at least one light emitting module 241. The at least one blocking member 270 may be configured to block light emitted from the at least one light emitting module 241 from propagating in the inner space of the housing 210. For example, the at least one blocking member 270 may be disposed adjacent to the light receiving module 242 in the inner space of the housing 210. For example, the at least one blocking member 270 may be positioned between the light receiving module 242 and the light emitting module 241 in the inner space of the housing 210.

FIG. 6A is a side cross-sectional view illustrating a wearable electronic device 200 according to embodiments of the disclosure. FIG. 6B is an enlarged view illustrating at least one protrusion C of the wearable electronic device 200 of FIG. 6A according to embodiments of the disclosure.

Referring to FIGS. 6A and 6B, the wearable electronic device 200 may include a housing 210 including an outer housing portion 211 and an inner housing portion 212, and at least one protrusion 213 disposed on the inner housing portion 212. The at least one protrusion 213 may be understood as a portion of a portion that directly contacts a user body in a state in which the user wears the wearable electronic device. When a plurality of protrusions 213 are provided, some protrusions may directly contact the user body, and other protrusions may not directly contact the user body. The configuration of the wearable electronic device 200 of FIG. 6 may be entirely or partially the same as the configuration of the wearable electronic device 200 of FIGS. 3 to 5. The embodiment of FIG. 6 may be partially combined with the embodiments of FIGS. 3 to 5.

According to an embodiment, the at least one protrusion 213 may press a blood vessel portion of a finger to better secure a blood vessel when emitting or receiving light to the blood vessel portion of the finger (e.g., when a light emitting module emits light). According to an embodiment, the at least one protrusion 213 may be disposed on the inner housing portion 212. According to an embodiment, the at least one protrusion 213 may constitute at least a portion of the inner housing portion 212. For example, when viewed in the state illustrated in FIG. 6A, the at least one protrusion 213 may be disposed to protrude toward the center O of the wearable electronic device 200 from the inner housing portion 212. According to an embodiment, the at least one protrusion 213 may be formed to protrude more inwardly from a surface of the inner housing portion 212 than other regions (e.g., portions where the protrusion is not disposed).

According to an embodiment, the at least one protrusion 213 may be integrally formed with the inner housing portion 212. For example, the at least one protrusion 213 may be a partial shape of the inner housing portion 212. The at least one protrusion 213 may be manufactured from a material such as a molding material for sensing, transparent plastic, or glass. For example, the at least one protrusion 213 may be configured to be at least partially transparent. For example, the at least one protrusion 213 may include a material through which light for measuring biometric data may be transmitted. For example, the transmissive material may be configured of a material through which at least a portion of the light emitted from the at least one light emitting module 241 for measuring biometric data is transmitted or through which at least a portion of the light reflected from a portion of the user's body for obtaining the biometric data is transmitted. A portion of the at least one protrusion 213 may be manufactured from substantially the same or similar material as the inner housing portion 212 and/or the outer housing portion 211.

According to an embodiment, a portion of the at least one protrusion 213 may include a metal material for biometric data measurement. For example, a sensor for obtaining biometric data may be implemented in a structure that directly applies an electrical signal to a user body or directly detects an electrical signal from the user body, in addition to the optical module 240 described above. In a sensor that directly applies or detects an electrical signal to/from a user body, a protrusion contacting the user body may function as an electrode terminal or an electrode pad.

According to an embodiment, a plurality of protrusions 213 may be disposed along one surface (e.g., an inner circumferential surface) of the inner housing portion 212. For example, when the plurality of protrusions 213 are provided, the protrusions 213 may be disposed along a circumferential direction (e.g., a circumferential direction) of the inner housing portion 212. For example, the plurality of protrusions 213 may be disposed in a first direction (e.g., a counterclockwise direction) and/or a second direction opposite to the first direction (e.g., a clockwise direction).

According to an embodiment, the at least one protrusion 213 may be formed such that a length from one surface of the inner housing portion 212 increases toward a central portion 2130 compared to an edge portion. As is described below, "the length of the protrusion from one surface of the inner housing portion 212 increases toward the central portion 2130" may be understood as the central portion 2130 being disposed closest to the center O of the wearable electronic device 200 on a surface of the protrusion 213. According to an embodiment, the central portion 2130 of the at least one protrusion 213 may be defined as a portion having the longest distance from one surface of the inner housing portion 212. According to an embodiment, the at least one protrusion 213 may be formed to protrude toward the center O of the wearable electronic device 200. According to an embodiment, a distance from the central portion 2130 of the at least one protrusion 213 to the center O of the wearable electronic device 200 may be shorter than a distance from an edge portion of the at least one protrusion 213 to the center O of the wearable electronic device 200. For example, a perpendicular distance from one surface of the inner housing portion 212 to the central portion 2130 of the at least one protrusion 213 may be a first distance h1. According to an embodiment, a perpendicular distance from one surface of the inner housing portion 212 to the edge portion of the at least one protrusion 213 may be a second distance h2. For example, the second distance h2 may be zero or greater than zero. For example, the second distance h2 may be formed to be shorter than the first length h1. According to an embodiment, the central portion 2130 of the at least one protrusion 213 may have a relatively large area in contact with a user's finger. According to an embodiment, the edge portion of the at least one protrusion 213 may have a relatively small area in contact with a user's finger or may not be in contact. For example, the central portion 2130 of the at least one protrusion 213 may substantially contact the user body.

According to an embodiment, the shape of the at least one protrusion 213 may vary. For example, the at least one protrusion 213 may be disposed on the inner housing portion 212, and one surface facing the center O of the wearable electronic device 200 may be a curved surface. For example, at least a portion of the central portion 2130 of the at least one protrusion 213 may be disposed substantially perpendicular to a reference line A passing through the center O of the wearable electronic device 200. "At least a portion of the central portion 2130 is disposed perpendicular to the reference line A passing through the center O of the wearable electronic device 200" may refer to a surface of the central portion 2130 or a tangent line at the central portion 2130 being aligned perpendicular to a straight line passing through the central portion 2130 and the center O. However, the shape of the at least one protrusion 213 is not limited to the embodiment, and at least a portion may form a flat surface and may be variously modified in design.

According to an embodiment, the at least one protrusion 213 may be disposed to correspond to a position where the optical module 240 (e.g., the light emitting module 241, the light receiving module 242) is disposed. Hereinafter, a structure and arrangement relationship of the optical module 240 and the at least one protrusion 213 is described.

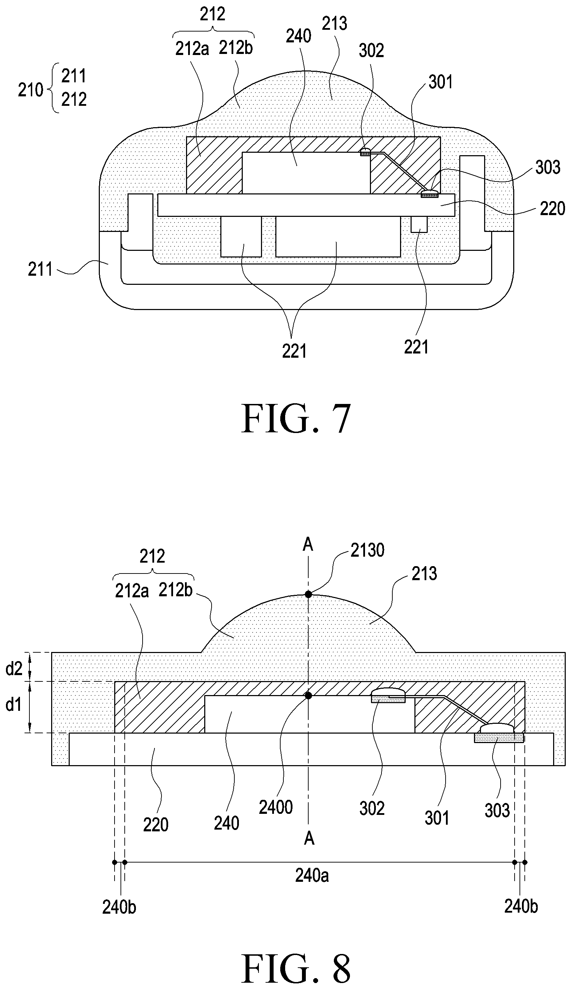

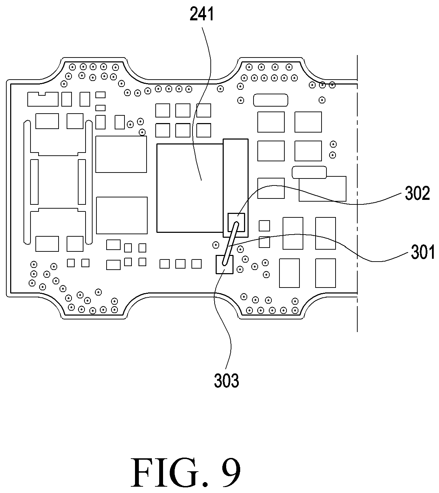

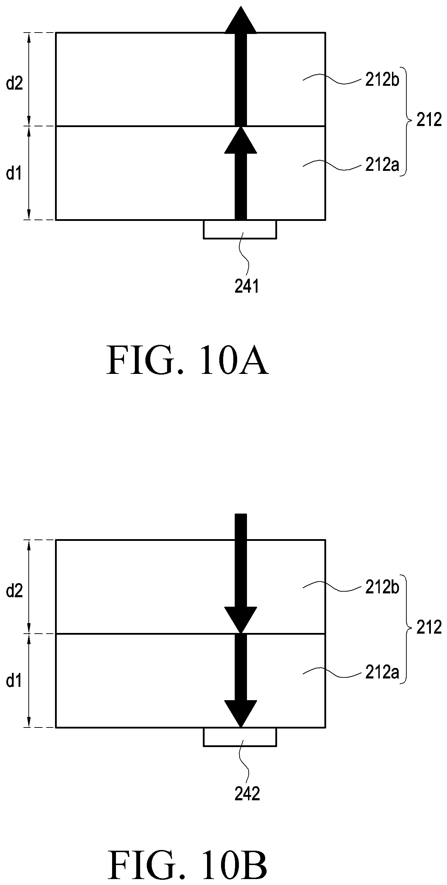

FIG. 7 is a side cross-sectional view illustrating a portion of a wearable electronic device according to embodiments of the disclosure. FIG. 8 is a side cross-sectional view illustrating an inner housing portion of a wearable electronic device according to embodiments of the disclosure. FIG. 9 is a view illustrating a circuit board 220 according to embodiments of the disclosure. FIGS. 10A and 10B are diagrams describing transmittance of a first coating layer 212a and a second coating layer 212b of an inner housing portion 212 according to embodiments of the disclosure.

Referring to FIGS. 7 to 9, the wearable electronic device 200 may include a housing 210 including an outer housing portion 211 and an inner housing portion 212, at least one protrusion 213 disposed on the inner housing portion 212, a circuit board 220, and an optical element 240. The configuration of the wearable electronic device 200 of FIGS. 7 to 9 may be entirely or partially the same as the configuration of the wearable electronic device 200 of FIG. 6. The embodiment of FIGS. 7 to 9 may be partially combined with the embodiment of FIG. 6.

According to an embodiment, the circuit board 220 may include at least one of a flexible printed circuit board (FPCB) or a rigid-flexible PCB (RF-PCB). According to an embodiment, various electrical/electronic components may be disposed and/or mounted on the circuit board 220. For example, an optical element 240 may be disposed on one surface of the circuit board 220 facing upward. For example, various components 221 (e.g., a processor (e.g., the processor 120 of FIG. 1), memory (e.g., the memory 130 of FIG. 1), a communication module (e.g., the communication module 190 of FIG. 1), or a sensor module (e.g., the sensor module 197 of FIG. 1)) may be disposed on one surface of the circuit board 220 facing downward.

According to an embodiment, the optical element 240 may be disposed on the circuit board 220. For example, the optical element 240 may include a light emitting element 241 (e.g., a light emitting diode (LED)) configured to emit light through the inner housing portion 212, and a light receiving element 242 (e.g., a photodiode (PD)) configured to receive light transmitted through or reflected from a user's finger. The optical element 240, the light emitting element 241, and the light receiving element 242 may be substantially the same as, or a component of, the optical module 240, the light emitting module 241, and the light receiving module 242 of FIG. 4.

According to an embodiment, the optical element 240 may be electrically connected to the circuit board 220. According to an embodiment, the optical element 240 (e.g., a photodiode) may be directly mounted on the circuit board 220 and electrically connected to the circuit board 220 through a conductor 301. The conductor 301 may include, e.g., a bonding wire. For example, the optical element 240 may be in a state not packaged with a substrate and another component (e.g., a semiconductor die).

According to an embodiment, when the non-packaged optical element 240 is directly mounted on the circuit board 220, the size of the optical element 240 may be relatively larger compared to a packaged element. A packaged element may have a relatively small size considering connection to other packaged components, but the non-packaged optical element 240 may use a larger area for the optical element 240 without a connection portion with other components. In this case, the amount of incident light increases, and a bio-signal may be received in a large amount. For example, a length in a width direction (X-axis direction) or length direction (Y-axis direction) of the optical element 240 may be about 2.0 mm. For example, a length in a height direction (Z-axis direction) of the optical element 240 may be about 2.5 mm or less. For example, an area of the optical element 240 may be about 4.0 mm².

According to an embodiment, by directly mounting the non-packaged optical element 240 on the circuit board 220 compared to a packaged element, an increase in size of the circuit board 220 or the wearable electronic device may be suppressed. In an embodiment, in being disposed in the same mounting space, the non-packaged optical element 240 may provide a larger light emitting region or light receiving region than a packaged structure. For example, a packaged element may have difficulty securing a light emitting region or a light receiving region in the same mounting space because a connection structure with other components is additionally required. According to embodiment(s) of the disclosure, by directly mounting the optical element 240 in a non-packaged state on the circuit board 220 to simplify a structure for connection to other components, a light emitting region or a light receiving region may be more widely secured.

According to an embodiment, the optical element 240 may include an active area 240a and an inactive area 240b extending from the active area 240a. According to an embodiment, when the non-packaged optical element 240 is directly mounted on the circuit board 220, the active area 240a of the optical element 240 may be larger compared to a packaged element. For example, when a width and length of the non-packaged optical element 240 are about 2 mm, a length in a width direction (X-axis direction) or length direction (Y-axis direction) of the active area 240a may be about 1.88 mm. For example, an area of the active area 240a may be about 3.53 mm².

According to an embodiment, when the non-packaged optical element 240 is directly mounted on the circuit board 220, a dedicated area ratio (e.g., an area of the active area 240a relative to a surface area of the optical element) may be larger compared to a packaged element. For example, the dedicated area ratio may be about 80% or more and 90% or less. For example, the dedicated area ratio may be about 88%. In the wearable electronic device 200 of limited size, light quantity may be enhanced and biometric measurement accuracy may be enhanced. Further, if the biometric measurement accuracy is the same, miniaturization in mounting of the optical element 240 is easy, which may be useful for miniaturization of the wearable electronic device 200, and design freedom inside the electronic device may be enhanced.

According to an embodiment, the optical element 240 may be electrically connected to the circuit board 220 through a conductor 301. The conductor 301 may be, e.g., any one of a wire, an electric wire, or an FPCB. According to an embodiment, the optical element 240 may contact the conductor 301 by a first adhesive member 302. For example, the first adhesive member 302 may be a silver paste (Ag paste) that may be cured even at a low temperature (about 100° C.). The circuit board 220 may contact the conductor 301 by a second adhesive member 303. For example, the first adhesive member 302 may be a silver paste (Ag paste) that may be cured even at a low temperature (about 100° C.). For example, a length of the conductor 301 may be about 0.01 mm or more and 0.03 mm or less. For example, the length of the conductor 301 may be about 0.017 mm. For example, the length of the conductor 301 may be about 0.5 mil or more and 1 mil or less. For example, the length of the conductor 301 may be about 0.7 mil. For example, a length in a width direction (X-axis direction) or length direction (Y-axis direction) of the first adhesive member 302 may be about 0.1 mil or more and 0.2 mil or less (e.g., about 0.002 mm or more and 0.006 mm or less). For example, a length in a width direction (X-axis direction) or length direction (Y-axis direction) of the first adhesive member 302 may be about 0.15 mil (e.g., about 0.003 mm or more and 0.004 mm or less). For example, a length in a width direction (X-axis direction) or length direction (Y-axis direction) of the second adhesive member 303 may be 0.1 mil or more and 0.3 mil or less (e.g., about 0.002 mm or more and 0.008 mm or less). For example, a length in a width direction (X-axis direction) or length direction (Y-axis direction) of the second adhesive member 303 may be 0.2 mil (e.g., about 0.005 mm or more and 0.006 mm or less). For example, a length in a width direction (X-axis direction) or length direction (Y-axis direction) of the second adhesive member 303 may be 0.15 mil (e.g., about 0.003 mm or more and 0.004 mm or less). For example, the second adhesive member 303 may be disposed at a distance separated by about 0.2 mil or more and 0.5 mil or less (e.g., about 0.005 mm or more and 0.02 mm or less) from the optical element 240. For example, the second adhesive member 303 may be disposed at a distance separated by about 0.3 mil (e.g., about 0.007 mm or more and 0.008 mm or less) from the optical element 240.

According to an embodiment, the inner housing portion 212 may have a structure in which a solution such as epoxy is cured to surround components disposed therein. For example, the inner housing portion 212 may have substantially no empty space or air gap therein. For example, after a process of assembling and/or coupling various components (e.g., a printed circuit board, a battery, an optical element) in the outer housing portion 211 is performed, the inner housing portion 212 may be formed through a process of injecting a molding solution with the outer housing portion inserted in a molding jig, and a curing process and/or a processing process.

According to an embodiment, the inner housing portion 212 may include a first coating layer 212a and a second coating layer 212b having different transmittances. The first coating layer 212a may be disposed to surround the optical element 240, and the light transmittance may be a first transmittance. The second coating layer 212b may be disposed to surround the first coating layer 212a, and the light transmittance may be a second transmittance lower than the first transmittance. Here, "(light) transmittance" may be defined as transmittance for light emitted from the light emitting element 241 or light to be detected through the light receiving element 242.

According to an embodiment, the first coating layer 212a and the second coating layer 212b may be formed from substantially the same solution, but there may be a difference in mixing concentration. For example, the first coating layer 212a and the second coating layer 212b may be formed from a solution such as epoxy. According to an embodiment, since the first coating layer 212a and the second coating layer 212b are formed from the same solution, the permittivity may be similar or substantially the same. Since the permittivity of the first coating layer 212a and the second coating layer 212b is substantially similar, a reflectance for light passing through a boundary between the first coating layer 212a and the second coating layer 212b may be low. For example, a reflected wave may not occur at the boundary between the first coating layer 212a and the second coating layer 212b. According to an embodiment, the first coating layer 212a disposed relatively inside may have a lower strength than the second coating layer 212b disposed relatively outside. For example, the strength of the second coating layer 212b forming a surface of the wearable electronic device may be relatively higher than the strength of the first coating layer 212a.

According to an embodiment, the first coating layer 212a may have a higher transmittance than the second coating layer 212b. According to an embodiment, when comparing the first coating layer 212a and the second coating layer 212b, the transmittance of the first coating layer 212a having relatively low strength may be relatively high, and the transmittance of the second coating layer 212b having relatively high strength may be relatively low. For example, the first transmittance of the first coating layer 212a may be about 90%, and the second transmittance of the second coating layer 212b may be about 80%. For example, the first transmittance of the first coating layer 212a may be about 95%, and the second transmittance of the second coating layer 212b may be about 90%.

According to an embodiment, referring to FIG. 10A, when a height of the first coating layer 212a is a first length d1 and a height of the second coating layer 212b is a second length d2, e.g., when the first transmittance of the first coating layer 212a is about 90% and the second transmittance of the second coating layer 212b is about 80%, light emitted from the light emitting element 241 may be transmitted by 90% by the first length d1 in the first coating layer 212a and transmitted by 80% by the second length d2 in the second coating layer 212b. For example, when the first length d1 and the second length d2 are substantially similar, an average transmittance may be 85%. For example, referring to FIG. 10B, when the first transmittance of the first coating layer 212a is about 95% and the second transmittance of the second coating layer 212b is about 90%, light received by the light receiving element 242 may be transmitted by 90% by the second length d2 in the second coating layer 212b and transmitted by 95% by the first length d1 in the first coating layer 212a. For example, when the first length and the second length are substantially similar, an average transmittance may be 92.5%.

According to an embodiment, by stacking and disposing the first coating layer 212a and the second coating layer 212b having different transmittances and strengths, both transmittance and strength may be enhanced.

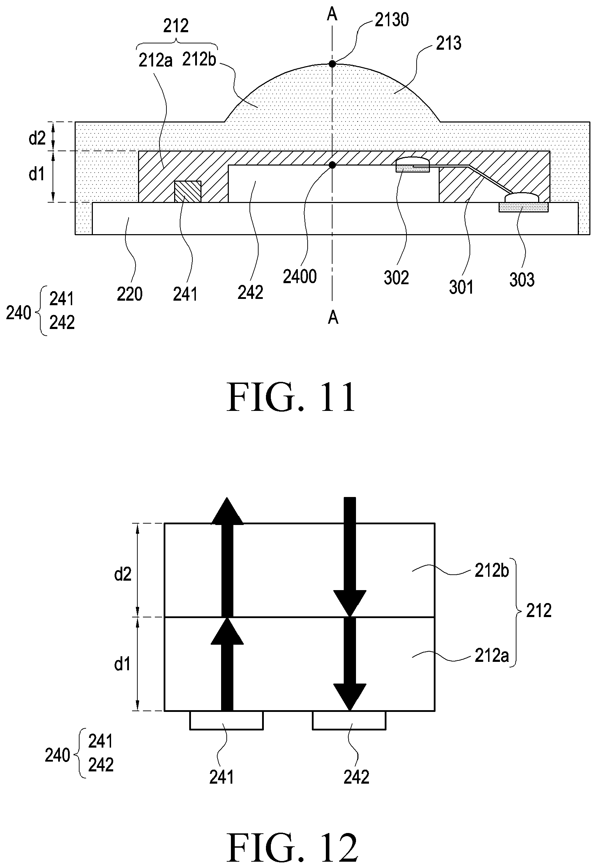

FIG. 11 is a side cross-sectional view illustrating an inner housing portion of a wearable electronic device in which both a light emitting element 241 and a light receiving element 242 are disposed according to embodiments of the disclosure. FIG. 12 is a diagram describing transmittance of a first coating layer 212a and a second coating layer 212b of an inner housing portion 212 according to embodiments of the disclosure.

Referring to FIGS. 11 and 12, the wearable electronic device 200 may include a housing 210 including an outer housing portion 211 and an inner housing portion 212, at least one protrusion 213 disposed on the inner housing portion 212, a circuit board 220, and an optical element 240. The configuration of the wearable electronic device 200 of FIGS. 11 and 12 may be entirely or partially the same as the configuration of the wearable electronic device 200 of FIGS. 7 to 9. The embodiment of FIGS. 11 and 12 may be partially combined with the embodiment of FIGS. 7 to 9.

According to an embodiment, the optical element 240 may be disposed on the circuit board 220. For example, the optical element 240 may include a light emitting element 241 (e.g., a light emitting diode (LED)) configured to emit light through the inner housing portion 212, and a light receiving element 242 (e.g., a photodiode (PD)) configured to receive light transmitted through or reflected from a user's finger. The optical element 240, the light emitting element 241, and the light receiving element 242 may be substantially the same as, or a component of, the optical module 240, the light emitting module 241, and the light receiving module 242 of FIG. 4.

According to an embodiment, the optical element 240 may be electrically connected to the circuit board 220. According to an embodiment, the optical element 240 (e.g., a photodiode) may be directly mounted on the circuit board 220 and electrically connected to the circuit board 220 through a conductor 301. The conductor 301 may include, e.g., a bonding wire. For example, the optical element 240 may be in a state not packaged with a substrate and another component (e.g., a semiconductor die).

According to an embodiment, the light emitting element 241 and the light receiving element 242 may be disposed adjacent to each other on the circuit board 220. The light emitting element 241 and the light receiving element 242 may be disposed side by side on the circuit board 220. According to an embodiment, referring to FIG. 11, the light receiving element 242 may be disposed to substantially correspond to the central portion 2130 of the at least one protrusion 213, and the light emitting element 241 may be disposed to correspond to an edge portion of the at least one protrusion 213. However, the arrangement of the light emitting element 241 and the light receiving element 242 is not limited by the embodiment and may be variously modified in design. For example, the light emitting element 241 may be disposed to correspond to the central portion 2130 of the at least one protrusion 213, and the light receiving element 242 may be disposed to correspond to an edge portion of the at least one protrusion 213. In this case, the light emitting element 241 that may be disposed adjacent to the light receiving element 242 may be, e.g., any one of a green LED chip, a red LED chip, a blue LED chip, and an infrared LED chip. The light emitting element 241 that may be disposed adjacent to the light receiving element 242 may be, e.g., a green LED chip.

According to an embodiment, the inner housing portion 212 may have a structure in which a solution such as epoxy is cured to surround components disposed therein. For example, the inner housing portion 212 may have substantially no empty space or air gap therein. For example, after a process of assembling and/or coupling various components (e.g., a printed circuit board, a battery, an optical element) in the outer housing portion 211 is performed, the inner housing portion 212 may be formed through a process of injecting a molding solution with the outer housing portion inserted in a molding jig, and a curing process and/or a processing process.

According to an embodiment, the first coating layer 212a may be disposed to surround both the light emitting element 241 and the light receiving element 242, and the light transmittance may be a first transmittance. The second coating layer 212b may be disposed to surround the first coating layer 212a, and the light transmittance may be a second transmittance lower than the first transmittance.

According to an embodiment, referring to FIG. 12, when the light emitting element 241 and the light receiving element 242 are disposed adjacent to each other, bi-directional transmission may be possible. When a height of the first coating layer 212a is a first length d1 and a height of the second coating layer 212b is a second length d2, e.g., when the first transmittance of the first coating layer 212a is about 90% and the second transmittance of the second coating layer 212b is about 80%, light emitted from the light emitting element 241 may be transmitted by 90% by the first length d1 in the first coating layer 212a and transmitted by 80% by the second length d2 in the second coating layer 212b. At the same time, light received by the light receiving element 242 may be transmitted by 80% by the second length d2 in the second coating layer 212b and transmitted by 90% by the first length d1 in the first coating layer 212a. For example, when the first length d1 and the second length d2 are substantially similar, an average transmittance may be 85%. According to an embodiment, by stacking and disposing the first coating layer 212a and the second coating layer 212b having different transmittances and strengths, both transmittance and strength may be enhanced.

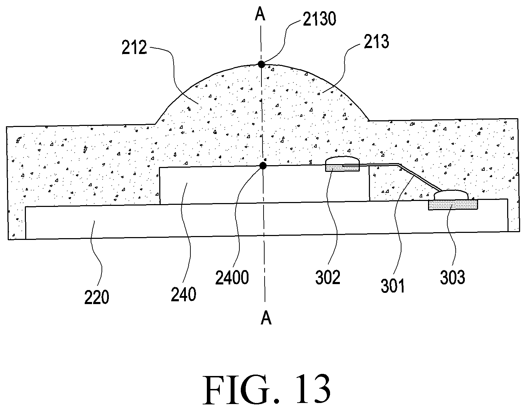

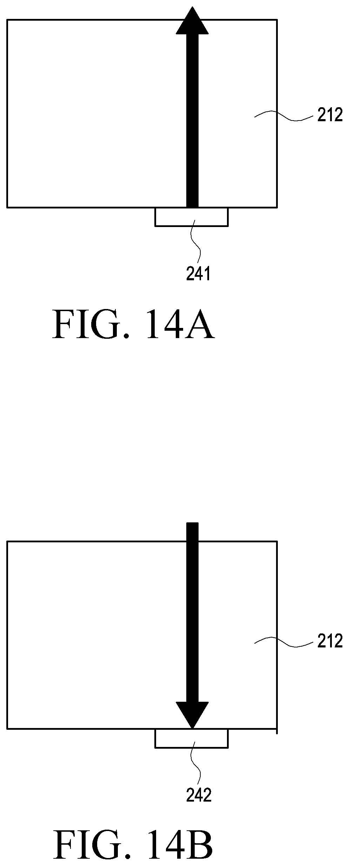

FIG. 13 is a side cross-sectional view illustrating a portion of a wearable electronic device including an inner housing portion 212 having substantially the same transmittance according to embodiments of the disclosure. FIGS. 14A and 14B are diagrams describing transmittance of an inner housing portion 212 according to embodiments of the disclosure.

Referring to FIGS. 13 to 14B, the wearable electronic device 200 may include a housing 210 including an outer housing portion 211 and an inner housing portion 212, at least one protrusion 213 disposed on the inner housing portion 212, a circuit board 220, and an optical element 240. The configuration of the wearable electronic device 200 of FIGS. 13 to 14B may be entirely or partially the same as the configuration of the wearable electronic device 200 of FIGS. 11 to 12. The embodiment of FIGS. 13 to 14B may be partially combined with the embodiment of FIGS. 11 to 12.

According to an embodiment, the optical element 240 may be disposed on the circuit board 220. For example, the optical element 240 may include a light emitting element 241 (e.g., a light emitting diode (LED)) configured to emit light through the inner housing portion 212, and a light receiving element 242 (e.g., a photodiode (PD)) configured to receive light transmitted through or reflected from a user's finger. The optical element 240, the light emitting element 241, and the light receiving element 242 may be substantially the same as, or a component of, the optical module 240, the light emitting module 241, and the light receiving module 242 of FIG. 4.

According to an embodiment, the inner housing portion 212 may be configured as a single layer. For example, the inner housing portion 212 may be configured of a first coating layer (e.g., 212a of FIG. 11) having a first transmittance. The inner housing portion 212 may be disposed to surround the optical element 240, and the light transmittance may be the first transmittance. For example, the inner housing portion 212 may be formed from a solution such as epoxy.

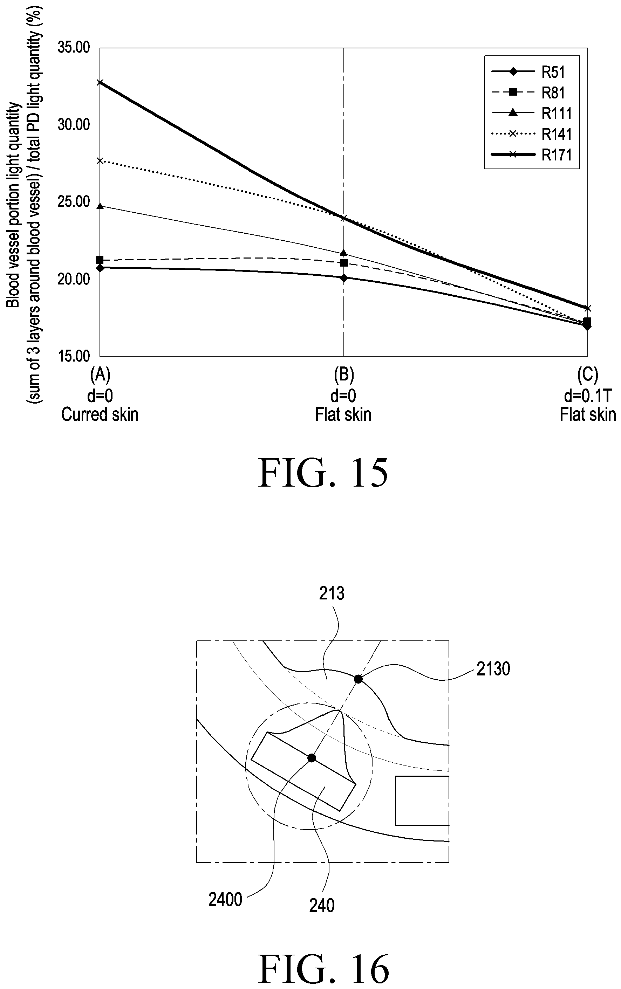

FIG. 15 is a view illustrating a change in light quantity at a blood vessel portion according to a curvature of at least one protrusion and a separation distance between the at least one protrusion and user skin according to embodiments of the disclosure. FIG. 16 is a side cross-sectional view illustrating distribution of light quantity entering an optical element 240 according to embodiments of the disclosure.

Referring to FIGS. 15 and 16, the wearable electronic device 200 may include a housing 210 including an outer housing portion 211 and an inner housing portion 212, at least one protrusion 213 disposed on the inner housing portion 212, a circuit board 220, and an optical element 240. The configuration of the wearable electronic device 200 of FIGS. 15 and 16 may be entirely or partially the same as the configuration of the wearable electronic device 200 of FIGS. 6 to 14. The embodiment of FIGS. 15 and 16 may be partially combined with the embodiment of FIGS. 6 to 14.

According to an embodiment, the central portion 2400 of FIG. 8 of the optical element 240, the central portion 2130 of FIG. 8 of the at least one protrusion 213, and the center O of the wearable electronic device 200 may be disposed substantially on a straight line to maximize a light quantity radiated to a blood vessel portion or a light quantity received by the light receiving element 242 after being reflected by (or transmitted through) the blood vessel portion. According to an embodiment, a curvature of the at least one protrusion 213 may increase from an edge portion toward the central portion 2130 of the at least one protrusion 213. Referring to FIG. 16, the larger the curvature of the at least one protrusion 213, the greater the amount of incident light may be. The amount of incident light may increase from the edge portion toward the central portion 2130 of FIG. 6A of the at least one protrusion 213. In other words, when the central portion 2400 of the optical element 240, the central portion 2130 of the at least one protrusion 213, and the center O of the wearable electronic device 200 are arranged along a straight line, the amount of incident light may increase, and an optical path may be formed at the central portion 2130 of the at least one protrusion 213 where no air gap occurs with user skin, which may be advantageous for obtaining light quantity at the blood vessel portion. When the amount of light emitted from the light emitting element 241 is the same, the narrower the region where the light quantity is radiated to the user body, and/or the larger the amount of light received by the light receiving element 242 after being transmitted through the user body or reflected by the user body, the higher the accuracy of obtaining or determining biometric data may be.

According to an embodiment, referring to FIG. 15, a change in blood vessel portion light quantity according to a separation distance d between the at least one protrusion 213 and user skin and a curvature R of the at least one protrusion 213 may be seen. According to an embodiment, (C) of FIG. 15 is a case where the separation distance d between the at least one protrusion 213 and user skin is 0.1T, and a protruding surface of the at least one protrusion 213 is flat. (B) of FIG. 15 is a case where the separation distance d between the at least one protrusion 213 and user skin is 0, and a protruding surface of the at least one protrusion 213 is flat. (A) of FIG. 15 is a case where the separation distance d between the at least one protrusion 213 and user skin is 0, and a protruding surface of the at least one protrusion 213 is curved. Each graph is a graph illustrating the curvature R of the at least one protrusion 213. Each graph illustrates a blood vessel portion light quantity/total received light quantity (%) when the curvature R of the at least one protrusion 213 is 51, 81, 111, 141, and 171.

According to an embodiment, in the case of (C) of FIG. 15, it may be identified that the blood vessel portion light quantity/total received light quantity (%) is very low at 20% or less regardless of the curvature R of the at least one protrusion 213. It may be identified that when the at least one protrusion 213 and user skin are separated, the blood vessel portion light quantity/total received light quantity (%) is low.

According to an embodiment, in the case of (B) of FIG. 15, it may be identified that the blood vessel portion light quantity/total received light quantity (%) is relatively higher compared to (C), and it may be identified that the lower the separation distance d between the at least protrusion 213 and user skin, the higher the blood vessel portion light quantity/total received light quantity (%).

According to an embodiment, in the case of (A) of FIG. 15, it may be identified that the blood vessel portion light quantity/total received light quantity (%) is relatively higher compared to (B), and it may be identified that when the at least one protrusion 213 is curved rather than when the at least one protrusion 213 is flat, the blood vessel portion light quantity/total received light quantity (%) is higher. For example, when the curvature R of the at least one protrusion 213 is 171, the blood vessel portion light quantity/total received light quantity (%) may be about 30% or more and 35% or less. For example, when the curvature R of the at least one protrusion 213 is 141, the blood vessel portion light quantity/total received light quantity (%) may be about 25% or more and 30% or less. For example, when the curvature R of the at least one protrusion 213 is 111, the blood vessel portion light quantity/total received light quantity (%) may be about 25%. For example, when the curvature R of the at least one protrusion 213 is 81, the blood vessel portion light quantity/total received light quantity (%) may be about 20% or more and 25% or less. For example, when the curvature R of the at least one protrusion 213 is 51, the blood vessel portion light quantity/total received light quantity (%) may be about 20%. In other words, it may be identified that the higher the curvature of the at least one protrusion 213, the higher the blood vessel portion light quantity/total received light quantity (%).

An electronic device according to an embodiment of the disclosure may include a housing 210 including an outer housing portion 211, and an inner housing portion 212 disposed inside the outer housing portion and including at least one protrusion 213 protruding in an inward direction, a circuit board 220 positioned within the housing, an optical element 240 positioned within the housing at a position corresponding to the at least one protrusion and mounted on the circuit board, and a conductor 301 electrically connecting the circuit board and the optical element. The inner housing portion may include a first coating layer 212a disposed to surround the optical element and having a first light transmittance.

According to an embodiment, the inner housing portion may further include a second coating layer 212b at least partially positioned on the at least one protrusion, disposed to surround the first coating layer, and having a second light transmittance lower than the first light transmittance.

According to an embodiment, the center O of the electronic device, the central portion 2130 of the at least one protrusion, and the central portion 2400 of the optical element may be arranged along a straight line.

According to an embodiment, the conductor may be any one of a wire, an electric wire, or a flexible printed circuit board (FPCB).

According to an embodiment, the first coating layer may have a lower strength than the second coating layer.

According to an embodiment, the electronic device may further include a processor disposed within the housing, the at least one optical element 240 may include a light emitting element 241 configured to emit light through the inner housing portion and a light receiving element 242 configured to receive at least a portion of the light transmitted through a user body or reflected by the user body after being emitted from the light emitting element, and the processor may be configured to obtain at least one type of biometric data using a signal sensed through the light receiving element.

According to an embodiment, the at least one type of biometric data may include at least one of oxygen saturation information and heart rate information.

According to an embodiment, the light receiving element may be disposed to correspond to the central portion of the at least one protrusion, the light emitting element may be disposed side by side with the light receiving element and disposed to correspond to an edge portion of the at least one protrusion, and the first coating layer may be disposed to surround the light emitting element and the light receiving element.

According to an embodiment, the at least one protrusion and the inner housing portion may be integrally formed.

According to an embodiment, the at least one protrusion may be configured to be at least partially transparent.

According to an embodiment, the circuit board may include a rigid circuit board and a flexible circuit board alternately disposed.

According to an embodiment, the electronic device may further include a battery 230 disposed within the housing, the battery may be disposed in a first region of the housing, and the circuit board may be disposed in a second region of the housing different from the first region.

According to an embodiment, the optical element may be configured to emit light in an inward direction of the housing via the central portion of the at least one protrusion.

According to an embodiment, the light emitting element may be configured to emit light of a plurality of wavelength bands, and the plurality of wavelength bands may include a green wavelength band, a red wavelength band, a blue wavelength band, and an infrared wavelength band.

According to an embodiment, the housing or the outer housing portion may have a ring shape.

An electronic device according to an embodiment of the disclosure may include a housing 210 including an outer housing portion 211, and an inner housing portion 212 disposed inside the outer housing portion, a circuit board 220 positioned within the housing, an optical element 240 positioned within the housing at a position corresponding to the at least one protrusion and mounted on the circuit board, and a conductor 301 electrically connecting the circuit board and the optical element. The inner housing portion may include a first coating layer 212a disposed to surround the optical element and having a first light transmittance, and a second coating layer disposed to surround the first coating layer and having a second transmittance lower than the first transmittance.

According to an embodiment, the inner housing portion may further include at least one protrusion protruding in an inward direction.

According to an embodiment, the center O of the electronic device, the central portion 2130 of the at least one protrusion, and the central portion 2400 of the optical element may be arranged along a straight line.

According to an embodiment, the electronic device may further include a processor disposed within the housing, the at least one optical element 240 may include a light emitting element 241 configured to emit light through the inner housing portion and a light receiving element 242 configured to receive at least a portion of the light transmitted through a user body or reflected by the user body after being emitted from the light emitting element, and the processor may be configured to obtain at least one type of biometric data using a signal sensed through the light receiving element.

An electronic device according to an embodiment of the disclosure may include a housing 210 including an outer housing portion 211, and an inner housing portion 212 disposed inside the outer housing portion and including at least one protrusion protruding in an inward direction, a circuit board 220 positioned within the housing, an optical element 240 positioned within the housing at a position corresponding to the at least one protrusion and mounted on the circuit board, and a conductor 301 electrically connecting the circuit board and the optical element. The inner housing portion may include a first coating layer 212a disposed to surround the optical element and having a first light transmittance, and a second coating layer disposed to surround the first coating layer and having a second transmittance lower than the first transmittance.

Technical problems to be achieved in this document are not limited to the technical problems mentioned above, and other technical problems not mentioned may be clearly understood by a person of ordinary skill in the technical field to which this document belongs from the following description.

A ring-type (finger ring-type) wearable electronic device wearable on a finger includes a healthcare function and has been increasing in market share recently. Since a ring-type wearable electronic device has a limited size, the size of a light receiving portion is also limited, which may cause a light receiving performance problem.

According to an embodiment of the disclosure, in a ring-type wearable electronic device of limited size, the size and the active area of the light receiving portion may be maximized, transmittance may be increased by stacking and disposing a plurality of materials having different physical properties, and an optical element, a protrusion 213, and the center of the electronic device may be arranged along a straight line to enhance a photo plethysmography (PPG) signal light quantity and increase light receiving efficiency. As the light receiving efficiency enhances, power consumption may be decreased to extend the usage time.

However, the objects of the disclosure are not limited to the foregoing objects but rather may be expanded in various manners without departing from the spirit and scope of the disclosure.

## Claims

1. An electronic device, comprising:
a housing (210) including an outer housing portion (211), and an inner housing portion (212) disposed inside the outer housing portion and including at least one protrusion (213) protruding in an inward direction;
a circuit board (220) positioned within the housing;
at least one optical element (240) positioned within the housing at a position corresponding to the at least one protrusion and mounted on the circuit board; and
a conductor (301) electrically connecting the circuit board and the at least one optical element,
wherein the inner housing portion includes a first coating layer (212a) disposed to surround the at least one optical element and having a first light transmittance.

2. The electronic device of claim 1, wherein the inner housing portion further includes a second coating layer (212b) at least partially positioned on the at least one protrusion, disposed to surround the first coating layer, and having a second light transmittance lower than the first light transmittance.

3. The electronic device of any one of claims 1 and 2, wherein a center (O) of the electronic device, a central portion (2130) of the at least one protrusion, and a central portion (2400) of the optical element are arranged along a straight line.

4. The electronic device of any one of claims 1 to 3, wherein the conductor is any one of a wire, an electric wire, or a flexible printed circuit board (FPCB).

5. The electronic device of claim 2, wherein the first coating layer has a lower strength than the second coating layer.

6. The electronic device of claim 3, further comprising a processor disposed within the housing, wherein the at least one optical element (240) includes:
a light emitting element (241) configured to emit light through the inner housing portion; and
a light receiving element (242) configured to receive at least a portion of the light transmitted through a user body or reflected by the user body after being emitted from the light emitting element, and
wherein the processor is configured to obtain at least one type of biometric data using a signal sensed through the light receiving element.

7. The electronic device of claim 6, wherein the at least one type of biometric data includes at least one of oxygen saturation information and heart rate information.

8. The electronic device of claim 6, wherein the light receiving element is disposed to correspond to the central portion of the at least one protrusion,
the light emitting element is disposed side by side with the light receiving element and disposed to correspond to an edge portion of the at least one protrusion, and
the first coating layer is disposed to surround the light emitting element and the light receiving element.

9. The electronic device of any one of claims 1 to 8, wherein the at least one protrusion and the inner housing portion are integrally formed.

10. The electronic device of any one of claims 1 to 9, wherein the at least one protrusion is configured to be at least partially transparent.

11. The electronic device of any one of claims 1 to 10, wherein the circuit board includes a rigid circuit board and a flexible circuit board alternately disposed.

12. The electronic device of any one of claims 1 to 11, further comprising a battery (230) disposed within the housing, wherein the battery is disposed in a first region of the housing, and the circuit board is disposed in a second region of the housing different from the first region.

13. The electronic device of claim 3, wherein the optical element is configured to emit light in an inward direction of the housing via the central portion of the at least one protrusion.

14. The electronic device of claim 6, wherein the light emitting element is configured to emit light of a plurality of wavelength bands, and the plurality of wavelength bands include a green wavelength band, a red wavelength band, a blue wavelength band, and an infrared wavelength band.

15. The electronic device of any one of claims 1 to 14, wherein the housing or the outer housing portion has a ring shape.
